# EUROPÄISCHE PATENTSCHRIFT

(11) **EP 0 701 831 B1**
(45) Veröffentlichungstag und Bekanntmachung des Hinweises auf die Patenterteilung: **11.11.1998**
(21) Anmeldenummer: 95114614.1
(22) Anmeldetag: 17.09.1995
(51) Int. Cl.: A61M 5/142

(54) **Implantierbare Infusionspumpe**
Implantable infusion pump
Pompe à perfusion implantable

(30) Priorität: 16.09.1994 DE 4432991
(43) Veröffentlichungstag der Anmeldung: 20.03.1996
(73) Patentinhaber: Fresenius AG, 61350 Bad Homburg v.d.H (DE)
(72) Erfinder: Steinbach, Bernd, Dr., D-61347 Bad Homburg v.d.H. (DE); Walter, Claus, D-61348 Bad Homburg v.d.H. (DE); Wild, Andreas, D-61168 Friedberg (DE)
(74) Vertreter: Luderschmidt, Schüler & Partner GbR

(56) Entgegenhaltungen:
- EP-A- 0 143 503
- DE-A- 2 604 113
- DE-A- 3 915 251

## Beschreibung

Die Erfindung betrifft eine implantierbare Infusionspumpe zur dosierten Abgabe von Medikamenten in den menschlichen Körper nach dem Oberbegriff des Anspruchs 1.

Eine derartige implantierbare Infusionspumpe ist aus der DE 39 15 251 A1 bekannt. Diese Infusionspumpe weist eine Pumpenkammer auf, die durch ein schalenförmiges Kammerunterteil und ein damit verbundenes Kammeroberteil gebildet ist. Die Pumpenkammer ist durch eine flexible Membran in zwei Teilkammern getrennt. Eine erste Teilkammer ist vom Kammeroberteil und der Membran begrenzt und als Medikamentenspeicher ausgebildet. Das Kammeroberteil enthält eine Nachfüllöffnung, die mit einem durchstechbaren Septum abgedichtet ist. Das Septum ist zwischen einem mit dem Kammeroberteil verbundenen Septenhalter und dem Kammeroberteil verklemmt. Der Medikamentenspeicher ist über eine Auslaßöffnung und ggf. eine Auslaßreduziereinrichtung mit einem Auslaßkatheter verbunden. Eine zweite Teilkammer wird vom Kammerunterteil und der Membran begrenzt und ist als Druckkammer zur Aufnahme eines sich bei Körpertemperatur ausdehnenden Treibmittels vorgesehen.

Ähnliche Infusionspumpen mit einem grundsätzlich gleichen Aufbau sind weiter bekannt aus der US-PS 3,731,681, der DE-OS 26 04 113 A1 sowie aus der US-PS 4,820,273.

Die Pumpenbauteile bestehen bei diesen Infusionspumpen aus körperverträglichen Metallegierungen oder Kunststoffen, die durch Schweißverbindungen oder einfache Rastverbindungen miteinander verbunden sind.

Derartige Infusionspumpen werden zur kontinuierlichen Medikation bei Patienten eingesetzt, die ansonsten nur durch Spritzen von Medikamenten mehrmals täglich behandelt werden könnten.

Die implantierbare Infusionspumpe ist dazu in einer subcutanen Tasche im Bereich des Abdomens angeordnet, wobei die mit dem Septum verschlossene Nachfüllöffnung unter der Haut des Patienten tastbar ist. Der Medikamentenspeicher wird gefüllt, indem eine Spritze mit einer entsprechenden Kanüle durch die Haut des Patienten und durch das Septum gestochen wird und das Medikament aufgrund des Spritzendrucks durch die Kanüle in den Medikamentenspeicher fließt.

An solche implantierbaren Infusionspumpen müssen hohe Sicherheitsanforderungen gestellt werden. Bei einem Bersten der Infusionspumpe durch Lösung der Verbindung einzelner Bauteile oder bei Undichtigkeiten an den Verbindungsstellen kommt es zu einem unkontrollierten Abfließen des Medikaments in den Körper des Patienten. Eine solche Überdosierung kann je nach verabreichtem Medikament zu einem hohen Gesundheitsrisiko führen.

Daher müssen die eingesetzten Verbindungen zwischen den Pumpenbauteilen für eine lange Verweilzeit von bis zu zehn Jahren ihre dichte Funktion beibehalten. Es kann jedoch durch eine unsachgemäße Befüllung des Medikamentenspeichers zu einer erheblichen Druckentwicklung kommen. Während der Betriebsdruck unter Normalbedingungen je nach verwendetem Treibmittel bei 0,6 bar bis 2,5 bar über Atmosphärendruck liegt, werden bei einer unsachgemäßen Befüllung Innendrücke in der Infusionspumpe von bis zu 6,5 bar über Atmosphärendruck gemessen. Damit treten erhebliche Überbelastungen insbesondere der Bauteilverbindungen auf mit der Gefahr, daß sich die Verbindungen lösen oder undicht werden mit den vorstehend geschilderten Risiken für einen Patienten.

Die DE-A-2604113 beschreibt eine implantierbare Vorrichtung zur dosierten Abgabe von Medikamenten, deren Gehäuse aus einem tassenförmigen unteren Gehäuseabschnitt und einem oberen Gehäuseabschnitt besteht, der ein zentrales Teil zur Aufnahme der Hauptkomponenten der Pumpe aufweist. Der untere und obere Gehäuseabschnitt sind miteinander verschweißt. Durch diese Schweißverbindung werden die Gehäuseteile fest miteinander verbunden. Zur Verbesserung der Körperverträglichkeit wird vorgeschlagen, das Gehäuse der Medikamentenpumpe mit einer elastischen Schicht zu überziehen, die aus einem für den menschlichen Körper verträglichen Material besteht.

Aufgabe der Erfindung ist es, die Verbindung von Bauteilen einer implantierbaren Infusionspumpe bei Innendrucküberlastungen sicherer zu gestalten.

Diese Aufgabe wird durch die kennzeichnenden Merkmale des Anspruchs 1 gelöst.

Bevorzugt werden die Haupt- und Nebenverbindungen durch Rastelemente als Rastverbindungen gebildet.

Somit wird zu einer Rastverbindung als Hauptverbindung eine weitere Rastverbindung als Nebenverbindung vorgesehen, die eine Reserveverhakung enthält. Diese Reserveverhakung greift weich und elastisch ein, wenn bei einer Überbelastung und Druckerhöhung in der Pumpe konstruktiv bedingte Bauteilformungen und Bauteilverschiebungen oder ein Versagen der Hauptverbindung auftreten.

Dadurch wird die Verbindung von Pumpenteilen sicherer gegen ein völliges Lösen der Verbindung und gegen Undichtigkeiten bei einer Drucküberlastung. Entsprechend werden die Risiken für einen Patienten reduziert. In einer bevorzugten Ausführungsform ist die Infusionspumpe als scheibenförmiger Körper mit abgerundeten Scheibenrändern aus Kunststoff ausgeführt. Die Rastverbindungen bestehen aus konzentrisch liegenden, umlaufenden Ringnuten und zugeordneten Ringstegen oder zugeordneten Rasthaken. Eine solche Ausführungsform ist fertigungstechnisch einfach herzustellen und die konstruktiv vorgesehenen Bauteilelastizitäten sind gut beherrschbar. Bevorzugt liegt die Nachfüllöffnung in an sich bekannter Weise zentrisch und das Septum ist als scheibenförmiges Zentralseptum ausgebildet. Am Kammeroberteil ist eine konzentrische Ringnut eingeformt, an deren innerer und äußerer Seitenwand jeweils umlaufend eine Rastnut angebracht ist. Der Septenhalter ist als Ring ausgebildet, der das Zentralseptum randseitig übergreift und der zwei in die Ringnut ragende, federnd nebeneinanderliegende Ringstege aufweist, die jeweils mit Rastnasen in die Rastnuten der Ringnut eingreifen. Die zwei Verbindungen an den beiden Rastnuten stellen eine Hauptverbindung und eine zugeordnete Nebenverbindung dar, wobei bevorzugt die äußere Rastverbindung die Hauptverbindung bildet.

In einer besonders bevorzugten Ausführungsform ist am oberen inneren Randbereich des schalenförmigen Kammerunterteils eine Ringnut vorgesehen, in die in einer Rastverbindung als Hauptverbindung ein umlaufender, zugeordneter Rand des Kammeroberteils zusammen mit einem O-Ring und dem Rand der Membran einrastbar ist. Am äußeren seitlichen Randbereich des Kammerunterteils ist eine weitere Ringnut vorgesehen, in die in einer Rastverbindung als zugeordnete Nebenverbindung eine Rastnasenanordnung, zweckmäßigerweise ein umlaufender Raststeg eines ringförmigen Überwurfteils eingreift. Das Überwurfteil übergreift dabei glockenförmig das Kammeroberteil und den äußeren seitlichen Randbereich des Kammerunterteils und stützt sich am Kammeroberteil ab. Damit sind das Kammerunterteil und Kammeroberteil sicher durch ein weiteres Bauteil in der Form eines Überwurfteils miteinander bei einer Drucküberlast verbunden. Dieses Überwurfteil kann vorzugsweise weiter dazu verwendet werden, einen abgedeckten Ringraum zur Aufnahme der Auslaßreduziereinrichtung und des Auslaßkatheteranschlusses zu bilden.

Bevorzugterweise ist an der Außenwand des Kommeroberteils eine Ringkammer angeordnet, die von einem Ringseptum abgedeckt und abgedichtet ist. Der innere Rand des Ringseptums ist vom äußeren Rand des ringförmigen Septenhalters überdeckt und verklemmt, während der ringäußere Rand des Ringseptums von einem Randbereich des Überwurfteils überdeckt und verklemmt ist. In diesem Randbereich des Überwurfteils ist eine Rastverbindung zwischen Überwurfteil und Kammeroberteil aus einer Rastnut und federnden Raststegen mit in die Rastnut eingreifenden Rastnasen vorgesehen. Zweckmäßig wird eine umlaufende Rastnut am Überwurfteil und eine umlaufende Rastnase am Kammeroberteil verwendet. Diese Rastverbindung stellt eine Hauptverbindung insbesondere zur Verklemmung und Abdichtung des Ringseptums dar. Die vorbeschriebene Rastverbindung zwischen Kammerunterteil und Überwurfteil bildet dazu eine Nebenverbindung.

Weitere Einzelheiten, Merkmale und Vorteile der Erfindung ergeben sich aus nachfolgender Beschreibung eines Ausführungsbeispiels anhand der Zeichnung.

Die einzige Figur zeigt einen Vertikalschnitt durch eine erfindungsgemäße, implantierbare Infusionspumpe.

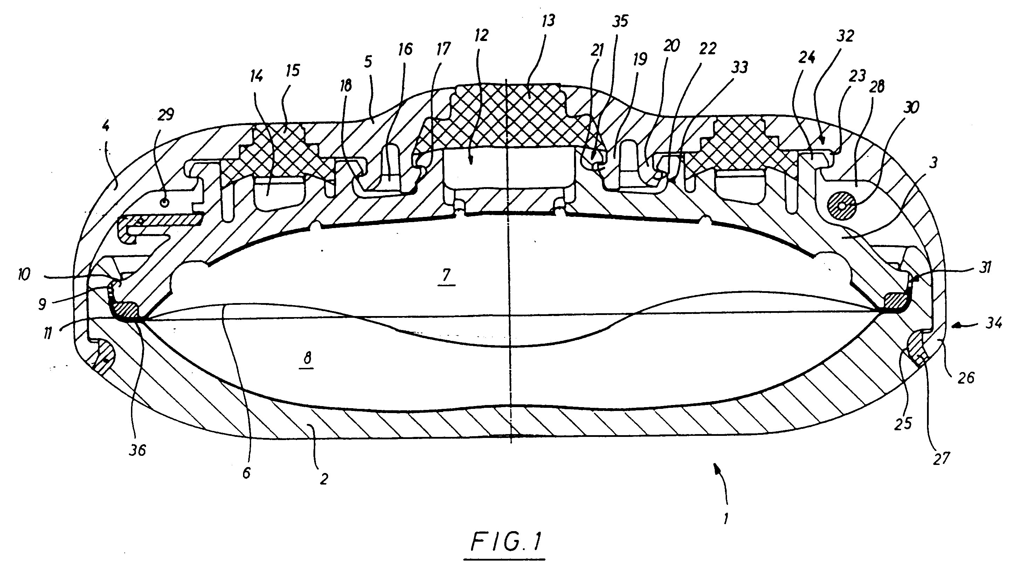

In Fig. 1 ist eine erfindungsgemäße, implantierbare Infusionspumpe 1 zur dosierten Abgabe von Medikamenten in den menschlichen Körper dargestellt. Die Infusionspumpe 1 ist ein scheibenförmiger rotationssymmetrischer Körper aus Kunststoff und weist ein Gehäuse auf, das aus einem schalenförmigen Kammerunterteil 2, einem schalenförmig mit entgegengesetzter Wölbung ausgeführten Kammeroberteil 3, einem Überwurfteil 4 und einem Septenhalter 5 zusammengesetzt ist. Der Innenraum ist durch eine flexible Membran 6 in eine erste Teilkammer als Medikamentenspeicher 7 und in eine zweite Teilkammer als Druckkammer 8 zur Aufnahme eines Treibmittels geteilt.

Am oberen inneren Randbereich des Kammerunterteils 2 ist eine Ringnut 9 eingeformt, in die in einer Rastverbindung ein umlaufender zugeordneter Rand 10 des Kammeroberteils 3 zusammen mit einem O-Ring 11 und einem Rand 36 der Membran 6 eingerastet und dicht gehalten ist.

Eine Nachfüllöffnung 12 ist durch ein scheibenförmiges und durchstechbares Zentralseptum 13 dicht abgedeckt, wobei die Nachfüllöffnung 12 einen Nachfüllraum unter dem Zentralseptum 13 mit einer festen Platte als Nadelstopp und Durchtrittsöffnungen zum Medikamentenspeicher 7 auweist.

Weiter ist an der Oberseite des Kammeroberteils 3 eine konzentrische Ringkammer 14 eingeformt, die von einem Ringseptum 15 dicht abgedeckt ist.

Zwischen der Ringkammer 14 und der Nachfüllöffnung 12 ist eine konzentrische Ringnut 16 eingeformt, an deren innerer und äußerer Seitenwand jeweils eine umlaufende Rastnut 17, 18 angebracht ist. Der ringförmige Septenhalter 5 übergreift mit seinem inneren Rand den Rand des scheibenförmigen Zentralseptums 13 und mit seinem äußeren Rand den ringinneren Rand des Ringseptums 15. Zudem greift der Septenhalter 5 mit nebeneinander liegenden Ringstegen 19, 20 in die Ringnut 16 ein, wobei Rastnasen 21, 22 auf den Ringstegen 19, 20 in die Rastnuten 17, 18 eingreifen.

Der ringäußere Rand des Ringseptums 15 ist in einer stufenförmigen Anordnung von einem entsprechend geformten Randbereich des Überwurfteils 4 überdeckt und gegen das Kammeroberteil 3 dicht verklemmt. Dazu ist in diesem Randbereich des Überwurfteils 4 eine Rastverbindung zwischen dem Überwurfteil 4 und dem Kammeroberteil 3 vorgesehen, die aus einer umlaufenden Rastnut 23 am Überwurfteil 4 und einer umlaufenden Rastnase 24 am Kammeroberteil 3 besteht.

Am seitlichen äußeren Randbereich des Kammerunterteils 2 ist eine weitere Ringnut 25 eingeformt, in die eine umlaufende Rastnase 26 zusammen mit einem eingelegten O-Ring 27 eingreift. Die Rastnase 26 stellt den unteren Rand des Überwurfteils 4 dar, wobei dieses den seitlichen Bereich des Kammeroberteils 3 und den äußeren seitlichen Randbereich des Kammerunterteils 2 glockenförmg übergreift. Das Überwurfteil 4 stützt sich (bei großer Druckbelastung) am Kammeroberteil 3 über die Rastnasenanordnung 24 und über das Ringseptum 15 zum Kammerunterteil 2 hin ab.

Zwischen dem äußeren Wandbereich des Kammeroberteils 3 und einem inneren Wandbereich des Überwurfteils 4 ist ein Ringraum 28 zur Aufnahme einer Auslaßreduziereinrichtung 29 (hier schematisch als Schnitt durch eine Kapillare dargestellt) und eines Auslaßkatheters 30 gebildet.

Die Verbindung 31, gebildet aus der Ringnut 9, dem Rand 10 und dem O-Ring 11, die Verbindung 32, gebildet aus der Rastnut 23 und der Rastnase 24 sowie die Verbindung 33 bestehend aus der Rastnut 18 und den Rastnasen 22 stellen Hauptverbindungen dar, die bei normaler betriebsmäßiger Belastung die auftretenden Innendruckbelastungen im wesentlichen abstützen.

Die Verbindung 34 bestehend aus der Ringnut 25, der Rastnase 26 und dem O-Ring 27 sowie die Verbindung 35 bestehend aus der Rastnut 17 und den Rastnasen 21 sind Nebenverbindungen, die bei normaler betriebsmäßiger Belastung noch keine oder nur eine geringe Abstützfunktion haben. Erst bei einer Druckerhöhung über den normalen Betriebsdruck, insbesondere bei einem Versagen der Hauptverbindungen 31, 32, 33 übernehmen die Nebenverbindungen 34, 35 eine Abstützfunktion.

Die Hauptverbindungen 31, 32, 33 stehen dazu in dauerndem Eingriff und stellen die Integrität der implantierten Infusionspumpe unter Normalbedingungen sicher. Bei Erhöhung des Innendrucks entweder im Ringraum 28 oder im Medikamentenspeicher 7 kommen die Nebenverbindungen 34, 35 zunehmend in Eingriff. Dadurch werden die Spannungen von den Hauptverbindungen 31, 32, 33 abgeleitet und teilen sich auf Haupt- und Nebenverbindungen auf.

Sollte beispielsweise die Hauptverbindung 31 versagen, so wird die Verbindungsaufgabe von der Nebenverbindung 34 übernommen. Da gleichzeitig ein Spalt zwischen Kammeroberteil 3 und Kammerunterteil 2 entsteht, entweicht Medikament in den Ringraum 28 zwischen dem Überwurfteil 4 und dem Kammeroberteil 3, so daß auch dadurch Druck und damit die Belastung auf die Verbindung insgesamt abnimmt. Dadurch ist die Nebenverbindung 34 in der Lage, die Integrität der Infusionspumpe nach außen hin sicherzustellen. Wesentlich ist dabei, daß das Unterteil im oberen Stegbereich versagt nicht aber im Unterbereich, was konstruktiv sichergestellt werden kann.

Bei einem Versagen der Hauptverbindung 32 zwischen dem Kammeroberteil 3 und dem Überwurfteil 4 wird die Verbindung ebenfalls durch die Nebenverbindung 34 übernommen.

Entsprechend wird bei einem Versagen oder einer Überlastung der Hauptverbindung 33 eine Übernahme oder Aufteilung auf die Nebenverbindung 35 übertragen.

In jedem Fall ergibt sich der Vorteil, daß bei einer Überlast oder bei einem Versagen der Hauptverbindungen 31, 32, 33 Nebenverbindungen 34, 35 zur Verfügung stehen, die den sicheren Weiterbetrieb der Infusionspumpe 1 gewährleisten, wodurch die Sicherheit für einen Patienten erheblich verbessert ist.

## Patentansprüche

1. Implantierbare Infusionspumpe (1) zu dosierten Abgabe von Medikamenten in den menschlichen Körper,
mit einer Pumpenkammer, die durch ein Kammerunterteil (2) und ein damit verbundenes Kammeroberteil (3) gebildet ist, wobei
die Pumpenkammer durch eine flexible Membran (6) in zwei Teilkammern unterteilt ist, deren erste Teilkammer vom Kammeroberteil (3) und der Membran (6) begrenzt ist und als Medikamentenspeicher (7) ausgebildet ist,
das Kammeroberteil (3) eine Nachfüllöffnung (12) enthält, die durch wenigstens ein durchstechbares Septum (13) abgedichtet ist, das zwischen einem mit dem Kammeroberteil (3) verbundenen Septenhalterteil (5) und dem Kammeroberteil (3) verklemmt ist,
der Medikamentenspeicher (7) über eine Auslaßöffnung und eine Auslaßreduziereinrichtung (29) mit einem Auslaßkatheter (30) verbunden ist, und
deren zweite Teilkammer vom Kammerunterteil (2) und der Membran (6) begrenzt ist und als Druckkammer (8) zur Aufnahme eines Treibmittels ausgebildet ist,
**dadurch gekennzeichnet,**
daß das Kammeroberteil (3), das Kammerunterteil (2) und das Septenhalterteil (5) derart ausgebildet sind, daß das Kammeroberteil (3) und das Kammerunterteil (2) sowie das Kammeroberteil (3) und das Septenhalterteil (5) über jeweils wenigstens zwei einander zugeordnete Verbindungen, von denen die eine als Hauptverbindung (31, 32, 33) und die andere als Nebenverbindung (34, 35) ausgeführt ist, in Eingriff sind,
wobei die Hauptverbindung (31, 32, 33) bei normaler betriebsmäßiger Belastung die auftretenden Innendruckbelastungen weitgehend abstützt und dabei in der Nebenverbindung (34, 35) noch keine oder nur eine geringe Abstützbelastung auftritt und bei einer durch eine Druckerhöhung über den normalen Betriebsdruck verursachten Verformung des Kammeroberteils (3), des Kammerunterteils (2) oder des Septenhalterteils (5) oder einer Verschiebung zwischen Kammeroberteil (3) und Kammerunterteil (2) sowie bei Versagen oder Nachgeben der Hauptverbindung (31, 32, 33) die Nebenverbindung (34, 35) entsprechend belastet wird und die Drucküberlast abstützt.

2. Implantierbare Infusionspumpe nach Anspruch, 1, dadurch gekennzeichnet, daß die einzelnen Verbindungen (31 - 35) durch Rastelemente als Rastverbindungen gebildet sind.

3. Implantierbare Infusionspumpe nach Anspruch 2, dadurch gekennzeichnet, daß die Infusionspumpe (1) als scheibenförmiger Körper mit abgerundeten Scheibenrändern aus Kunststoff ausgeführt ist und die Rastverbindungen als Haupt- und Nebenverbindungen (31 - 35) mit konzentrisch liegenden umlaufenden Ringnuten und zugeordneten Ringstegen oder Rasthaken ausgeführt sind.

4. Implantierbare Infusionspumpe nach Anspruch 3, dadurch gekennzeichnet,
daß die Nachfüllöffung (12) zentrisch und das Septum als scheibenförmiges Zentralseptum (13) ausgebildet sind,
daß am Kammeroberteil (3) eine konzentrische Ringnut (16) eingeformt ist, an deren innerer und äußerer Seitenwand jeweils umlaufend eine Rastnut (17, 18) angebracht ist,
daß das Septenhalterteil (5) als Ring ausgebildet ist, der das Zentralseptum (13) randseitig übergreift und der zwei in die Ringnut (16) ragende, nebeneinander liegende Ringstege (19, 20) aufweist, die jeweils mit Rastnasen (21, 22) in die Rastnuten (17, 18) der Ringnut (16) eingreifen, wobei die zwei Verbindungen an den beiden Rastnuten (17, 18) eine Hauptverbindung (33) und eine zugeordnete Nebenyerbindung (35) darstellen.

5. Implantierbare Infusionspumpe nach Anspruch 4, dadurch gekennzeichnet, daß die Verbindung an der äußeren Rastnut (18) die Hauptverbindung (33) und die Verbindung an der inneren Rastnut (19) die Nebenverbindung (35) bildet.

6. Implantierbare Infusionspumpe nach einem der Ansprüche 2 bis 5, dadurch gekennzeichnet,
daß am oberen, inneren Randbereich des schalenförmigen Kammerunterteils (2) eine Ringnut (9) vorgesehen ist, in die in einer Rastverbindung als Hauptverbindung (31) ein umlaufender zugeordneter Rand (10) des Kammeroberteils (3) zusammen mit einem O-Ring (11) und dem Rand (12) der Membran (6) eingerastet ist,
daß am äußeren seitlichen Randbereich des Kammerunterteils (2) eine weitere Ringnut (25) vorgesehen ist, in die in einer Rastverbindung als zugeordnete Nebenverbindung (34) eine Rastnasenanordnung (26) eines ringförmigen Überwurfteils (4) eingreift, wobei das Überwurfteil (4) das Kammeroberteil (3) und den äußeren seitlichen Randbereich des Kammerunterteils (2) glockenförmig übergreift und sich am Kammeroberteil (3) abstützt.

7. Implantierbare Infusionspumpe nach Anspruch 6, dadurch gekennzeichnet, daß zwischen einem äußeren Wandbereich des Kammeroberteils (3) und einem inneren Wandbereich des überwurfteils (4) ein Ringraum (28) gebildet ist.

8. Implantierbare Infusionspumpe nach Anspruch 7, dadurch gekennzeichnet, daß der Ringraum zur Aufnahme der Auslaßreduziereinrichtung (29) und des Auslaßkatheters (30) gebildet ist.

9. Implantierbare Infusionspumpe nach einem Ansprüche 6 bis 8, dadurch gekennzeichnet,
daß an der Außenwand des Kammeroberteils (3) eine die Ringnut (16) umgebende Ringkammer (14) angeordnet ist, die von einem Ringseptum (15) abgedeckt und abgedichtet ist,
daß der zur Ringnut (16) weisende Rand des Ringseptums (15) von einem äußeren Rand des ringförmigen Septenhalterteils (5) überdeckt und verklemmt ist,
daß der ringäußere Rand des Ringseptums (15) von einem Randbereich des Überwurfteils (4) überdeckt und verklemmt ist, und
daß in diesem Randbereich des Überwurfteils (4) eine Rastverbindung (32) zwischen Überwurfteil (4) und Kammeroberteil (3) aus einer Rastnut (23) und Raststegen mit in die Rastnut (23) eingreifenden umlaufenden Rastnasen (24) vorgesehen ist, bevorzugt eine umlaufende Rastnut (23) am Überwurfteil (4) und eine umlaufende Rastnase (24) am Kammeroberteil (3), und
daß diese Rastverbindung (32) eine Hauptverbindung darstellt und daß die Rastverbindung (34) zwischen Kammerunterteil (2) und Überwurfteil (4) dazu eine Nebenverbindung bildet.

## Claims

1. Implantable infusion pump (1)for the dosed administration o medications into the human body,
with a pump chamber, which is formed by a lower chamber section (2) and an upper chamber section (3) connected to it, in which situation
the pump chamber is subdivided by a flexible membrane (6) into two part chambers, of which the first part chamber is delimited by the upper chamber section (3) and the membrane (6), and is designed as a medication storage space (7),
the upper chamber section (3) contains a refilling aperture (12), which is sealed by at least one perforable septum (13), which is clamped between a septum holder (5), which is connected to the upper chamber section (3), and the upper chamber section (3),
the medication storage space (7), connected via an outlet aperture and an outlet reducer device (29) to an outlet catheter (30), and
the second part chamber of which is delimited by the lower chamber section (2) and the membrane (6), and is designed as a pressure chamber (8) for accommodating a propellant agent,
characterised in that
the upper chamber section (3), the lower chamber section (2), and the septum holder element (5) are designed in such a way that the upper chamber section (3) and the lower chamber section (2), as well as the upper chamber section (3) and the septum holder element (5) are engaged in each case by means of at least two connections arranged in mutual opposition t one another, of which one is designed as the main connection (31, 32, 3) and the other as an ancillary connection (34, 35),
in which situation the main connection (31, 32, 33) under normal operational load largely supports the internal pressure loads which occur, and in which situation the ancillary connection (34, 35) still incurs no or only a slight support load, and, in the event of deformation of the upper chamber section (3), of the lower chamber section (2), or the septum holder section (5), caused by a pressure increase beyond the normal operational pressure, or a displacement between the upper chamber section (3) and the lower chamber section (2), and in the event of the failure or yielding of the main connection (31, 32, 33), the ancillary connection (34, 35) will be loaded accordingly and will support the pressure overload.

2. Implantable infusion pump according to Claim 1, characterised in that the individual connections (31 - 35) are formed as engagement connections by means of engagement elements.

3. Implantable infusion pump according to Claim 2, characterised in that the infusion pump (1) is designed as a disk-shaped body with rounded disk edges, made of plastic, and the engagement connections are designed as main and ancillary connections (31 -35) with concentric circumferential ring grooves and ring projections or engagement hooks associated with them.

4. Implantable infusion pump according to Claim 3, characterised in that
the refilling aperture (12) is designed centrically and the septum is designed as a disk-shaped central septum (13),
a concentric ring groove (16) is formed on the upper chamber section (3), on the inner and outer side walls of which, in each case, an engagement groove (17, 18) is arranged,
the septum holder section (5) is designed as a ring, which engages over the central septum (13) on the peripheral side, and which features two ring projections (19, 20) located next to one another, which project into the ring groove (16), these engaging in each case by means of engagement lugs (21, 22) into the engagement groves (17, 18) of the ring groove (16), in which situation the two connections on the two engagement grooves (17, 18) represent a main connection (33) and an associated ancillary connection (35).

5. Implantable infusion pump according to Claim 4, characterised in that he connection at the outer engagement groove (18) forms the main connection (33) and the connection at the inner engagement groove (19) forms the ancillary connection (35).

6. Implantable infusion pump according to one of Claims 2 to 5, characterised in that
on the upper, inner peripheral area of the disk-shaped lower chamber section (2) a ring groove (9) is provided, into which engages, in an engagement connection as a main connection (13), a circumferential periphery (10) of the upper chamber section (3) is allocated to the groove, together with an O-ring (11) and the periphery (12) of the membrane,
on the outer lateral periphery area of the lower chamber section (2) another ring groove (25) is provided, into which engages, in an engagement connection as an allocated ancillary connection (34), an engagement lug arrangement (26) of a ring-shaped locking component (4), in which situation the locking component (4) engages in a bell shape over the upper chamber section (3) and the outer lateral peripheral area of the lower chamber section (2), and is supported on the upper chamber section (3).

7. Implantable infusion pump according to Claim 6, characterised in that a ring space (28) is formed between one outer wall area of the upper chamber section (3) and an inner wall area of the locking component (4).

8. Implantable infusion pump according to Claim 7, characterised in that the ring space is formed to accommodate the outlet reducer device (29) and the outlet catheter (30).

9. Implantable infusion pump according to one of Claims 6 to 8, characterised in that
on the outer wall of the upper chamber section (3) a ring chamber (14) is arranged, surrounding the ring groove (16), which is covered and sealed by a ring septum (15),
the periphery of the ring septum (15) which faces the ring groove (16) is covered and clamped by an outer edge of the ring-shaped septum holder section (5),
the outer ring periphery of the ring septum (15) is covered and clamped by a peripheral section of the locking component (4), and
that in this peripheral area of the locking component (4) an engagement connection (32) is provided between the locking component (4) and the upper chamber section (3), from an engagement groove (23) and engagement projections with circumferential engagement lugs (24) engaging in the engagement groove (23), for preference a circumferential engagement groove (23) at the locking component (4) and a circumferential engagement lug (24) at the upper chamber section (3), and
this engagement connection (32) represents a main connection and that the engagement connection (34) between the lower chamber section (2) and the locking component (4) forms an ancillary connection thereto.

## Revendications

1. Pompe à perfusion implantable (1), destinée à instiller des doses de médicaments dans le corps humain, comprenant une chambre, qui est formée par une partie inférieure (2) et une partie supérieure (3) reliée à ladite partie inférieure,
la chambre de la pompe étant séparée en deux chambres partielles par une membrane flexible (6), dont la première chambre partielle est délimitée par la partie supérieure de la chambre (3) et par la membrane (6) et est conçue comme un réservoir à médicaments (7),
la partie supérieure de la chambre (3) étant munie d'un orifice de remplissage (12), qui est rendu étanche par au moins un septum (13) susceptible d'être perforé, et qui est bloqué entre un porte-septum (5), assemblé à la partie supérieure de la chambre (3), et la partie supérieure de la chambre (3),
le réservoir à médicaments (7) étant relié à un cathéter d'instillation (30) par l'intermédiaire d'un orifice d'évacuation et d'un dispositif de réduction de l'instillation (29), et
dont la deuxième chambre partielle est délimitée par la partie inférieure de la chambre (2) et la membrane (6) et est conçue sous forme de chambre de compression (8) destinée à recevoir un fluide moteur,
caractérisée en ce que
la partie supérieure de la chambre (3), la partie inférieure de la chambre (2) et le porte-septum (5) sont conçus de telle sorte que la partie supérieure de la chambre (3) et la partie inférieure de la chambre (2), ainsi que la partie supérieure de la chambre (3) et le porte-septum (5) sont assemblés respectivement par deux assemblages correspondants l'un à l'autre, dont l'un constitue un assemblage principal (31, 32, 33) et l'autre forme un assemblage secondaire (34, 35),
l'assemblage principal (31, 32, 33) dans des conditions de sollicitations normales supporte largement les sollicitations de pression interne qui apparaissent et, à cette occasion, l'assemblage secondaire (34, 35) n'exerçant pas ou seulement une faible fonction de support et, en cas d'une déformation de la partie supérieure de la chambre (3), de la partie inférieure de la chambre (2) ou du porte-septum (5) ou en cas de déplacement entre la partie supérieure de la chambre (3) et la partie inférieure de la chambre (2), dus à une augmentation de la pression supérieure à la pression de service normale, ainsi qu'en cas de défaillance ou de desserrement de l'assemblage principal (31, 32, 33), l'assemblage secondaire (34, 35) est sollicité de manière correspondante et supporte la sollicitation de pression excessive.

2. Pompe à perfusion implantable selon la revendication 1, caractérisée en ce que chaque assemblage (31 - 35) est formé par des éléments à encastrer constituant un assemblage par encastrement.

3. Pompe à perfusion implantable selon la revendication 2, caractérisée en ce que la pompe à perfusion (1) est un corps en matière plastique, en forme de disque avec des arêtes arrondies, et les assemblages par encastrement sont exécutés sous forme d'assemblages principaux et d'assemblages secondaires (31 - 35) avec des gorges annulaires périphériques et concentriques et des nervures annulaires ou des talons de blocage correspondants.

4. Pompe à perfusion implantable selon la revendication 3, caractérisée
en ce que l'orifice de remplissage (12) est situé au centre et le septum est conçu sous forme de septum central (13) en forme de disque,
en ce qu'une gorge annulaire (16) concentrique est moulée dans la partie supérieure de la chambre (3) et une gorge de blocage (17, 18) périphérique est réalisée respectivement sur la paroi latérale intérieure et êxtérieure de ladite gorge annulaire,
en ce que le porte-septum (5) est conçu sous forme de bague, qui bloque le bord du septum central (13) et qui est muni de deux nervures annulaires (19, 20) faisant saillie dans la gorge annulaire (16), lesquelles sont disposées de manière élastique l'une à côté de l'autre, s'encastrent avec des talons de blocage (21, 22) dans les gorges de blocage (17, 18) de la gorge annulaire (16), les deux assemblages au niveau des deux gorges de blocage (17, 18) constituant un assemblage principal (33) et un assemblage secondaire (35) correspondant.

5. Pompe à perfusion implantable selon la revendication 4, caractérisée en ce que l'assemblage au niveau de la gorge de blocage extérieure (18) constitue l'assemblage principal (33) et l'assemblage au niveau de la gorge de blocage intérieure (19) constitue l'assemblage secondaire (35).

6. Pompe à perfusion implantable selon l'une quelconque des revendications 2 à 5, caractérisée
en ce qu'il est prévu de réaliser, sur le bord supérieur intérieur de la partie inférieure de la chambre (2) en forme de coque, une gorge annulaire (9), dans laquelle s'encastrent le bord périphérique (10) correspondant de la partie supérieure de la chambre (3), ainsi qu'un joint torique (11) et le bord (12) de la membrane (6), pour former un assemblage par encastrement constituant un assemblage principal (31),
en ce qu'il est prévu de réaliser, sur le bord latéral extérieur de la partie inférieure de la chambre (2), une autre gorge annulaire (25), dans laquelle s'encastre un agencement à talons de blocage (26) réalisé sur une pièce d'accouplement annulaire (4), pour former un assemblage par encastrement constituant un assemblage secondaire (34), la pièce d'accouplement (4) s'engageant en forme de cloche au-dessus de la partie supérieure de la chambre (3) et du bord latéral extérieur de la partie inférieure de la chambre (2) et s'appuyant contre la partie supérieure de la chambre (3).

7. Pompe à perfusion implantable selon la revendication 6, caractérisée en ce que, entre la paroi extérieure de la partie supérieure de la chambre (3) et la paroi intérieure de la pièce d'accouplement (4), est réalisé un espace annulaire (28).

8. Pompe à perfusion implantable selon la revendication 7, caractérisée en ce que l'espace annulaire est conçu pour recevoir un dispositif de réduction de l'instillation (29) et un cathéter d'instillation (30).

9. Pompe à perfusion implantable selon l'une quelconque des revendications 6 à 8, caractérisée
en ce qu'une chambre annulaire (14) est disposée au niveau de la paroi extérieure de la partie supérieure de la chambre (3), laquelle chambre annulaire est protégée et rendue étanche par un septum annulaire (15),
en ce que le bord intérieur du septum annulaire (15), orienté vers la gorge annulaire (16), est recouvert et bloqué par un bord extérieur du porte-septum (5) annulaire,
en ce que le bord extérieur annulaire du septum annulaire (15) est recouvert et bloqué par une zone de bordure de la pièce d'accouplement (4),
en ce que, dans ladite zone de bordure de la pièce d'accouplement (4), il est prévu un assemblage par encastrement (32) entre la pièce d'accouplement (4) et la partie supérieure de la chambre (3), lequel assemblage est formé par une gorge de blocage (23) et des nervures de blocage avec des talons de blocage (24) s'encastrant dans la gorge de blocage (23), de préférence, une gorge de blocage (23) périphérique sur la pièce d'accouplement (4) et un talon de blocage (24) périphérique sur la partie supérieure de la chambre (3),
en ce que cet assemblage par encastrement (32) constitue un assemblage principal et en ce que l'assemblage par encastrement (34), entre la partie inférieure de la chambre (2) et la pièce d'accouplement (4), constitue un assemblage secondaire correspondant.
